# EUROPEAN PATENT SPECIFICATION

(11) **EP 2 347 702 B1**
(45) Date of publication and mention of the grant of the patent: **16.07.2014**
(21) Application number: 10290034.7
(22) Date of filing: 22.01.2010
(51) Int. Cl.: A61B 3/16

(54) **Multi-diagnostic contactless system using ocular physiological parameters**
Kontaktloses Multidiagnosesystem unter Verwendung physiologischer Okularparameter
Système multidiagnostic sans contact utilisant des paramètres physiologiques oculaires

(43) Date of publication of application: 27.07.2011
(73) Proprietor: Ophtimalia, 14460 Colombelles (FR)
(72) Inventor: Auvray, Philippe, 14000 Caen (FR); Cauvet, Philippe, 14000 Caen (FR); Verjus, Fabrice, 14480 Creully (FR); Biemans, Peter, 14112 Biéville-Beuville (FR)
(74) Representative: Grünecker, Kinkeldey, Stockmair & Schwanhäusser

(56) References cited:
- EP-A2- 0 061 777
- WO-A1-2007/136993
- WO-A2-03/073959

## Description

The invention relates to a physiological parameter measuring system, in particular for measuring the intraocular eye pressure (IOP), comprising a contact lens. The invention also relates to a corresponding method.

Intraocular pressure is a physiological parameter used to diagnose glaucoma, which is characterized by an elevated pressure with, in many cases, large variations inside the eye.

Typically, the intraocular pressure is measured using special ophthalmological devices called Tonometers. The measurements can only be carried out in a doctor's practice or at hospital. As a consequence, this kind of measurement needs the presence of the patient in the practice or at hospital and the IOP pressure is only measured at a given time during the day and no information about the evolution of the IOP is obtained during a long period of the day in a patient's normal environment.

There is, therefore, a need to provide an improved measuring and monitoring system that will enable to record possible causes, comprising physical activities, environmental, posture or psychological causes that may have an effect on the variations of the IOP value. At the same time, knowing at any time the value of the physiological parameter under study, it will become possible for patients to not only obtain an improved diagnostic but it will also become possible to follow an adapted medical treatment.

EP 061 777 discloses a system to measure the inter-ocular eye pressure using a sensor comprising a contact lens and a conductive spiral on its front and backside arranged around a protrusion in the centre of the contact lens towards a patient's eye. The system is completed by spectacles carrying an antenna for sensing the resonance properties of an LC circuit formed by the spirals on the contact lens positioned on a patient's eye. Based on the shift of the resonance frequency of the LC circuit information about the patient's eye pressure can be obtained.

Prior art document WO 03/001991 proposes an intraocular pressure recording system allowing a continuous monitoring over prolonged periods, regardless of a patient's position and activities. The recording system described therein comprises a soft contact lens including an active strain gauge for measuring spherical deformations fixed to one side of the contact lens. The information is transmitted to an external recording system.

EP 061 777 discloses a system to measure the inter-ocular eye pressure using a sensor comprising a contact lens and a conductive spiral on its front and backside arranged around a protrusion in the centre of the contact lens towards a patient's eye. The system is completed by spectacles carrying an antenna for sensing the resonance properties of an LC circuit formed by the spirals on the contact lens positioned on a patient's eye. Based on the shift of the resonance frequency of the LC circuit information about the patient's eye pressure can be obtained.

There is a need to improve such an intraocular pressure recording system.

This object is achieved with the physiological parameter measuring system according to claim 1 comprising one or two contact lenses, wherein each contact lens comprises an antenna and a purely passive sensing means. The antenna and the purely passive sensing means comprise spires on the on the one side of a flexible carrier substrate and spires on the other side of a flexible carrier substrate, wherein the spires are connected via a plurality of vias with each other. This arrangement reduces the resistivity of the circuit and thus improves the quality factor of the LC circuit.

Preferably, the physiological parameter is the intraocular eye pressure.

Preferably, the passive sensing means can comprise a capacitor or a transducer, in particular a piezoelectric transducer. Using a capacitor, which can be an off the shelf capacitor or fabricated together with the antenna before being embedded into the contact lens, and thus leads to a simplified design representing a LC circuit. A piezoelectric transducer can replace the capacitor and is arranged in parallel with the antenna. The electrical equivalent model shows that the principle of measuring the variation of the resonant frequency is still valid. This solution offers a higher sensitivity, thus less energy is required to power the sensor. Using this kind of sensing means, unwanted effects of radiation and of energy dissipation, which might be harmful to the patient, can be lowered. At the same time, variations of the physiological parameter can be identified based on the observed resonant frequency shift.

Advantageously, the physiological parameter measuring system can further comprise one or two antenna patches external to the one or two contact lenses. Via the antenna patches an inductive coupling can be achieved with the circuitry of the contact lens so that signals provided by the sensing means can be received by the antenna patch via the antenna of the contact lens. As no cable connection is necessary, the patient's comfort during a measurement series of the IOP is improved compared to measuring systems which are cable based.

Advantageously, the antenna patch can comprise a transparent, in particular flexible, support layer and an adhesive layer. This antenna patch can thus be simply attached to a pair of spectacles, so that they can be positioned in front of the contact lens to ensure an inductive coupling between the antenna of the contact lens and the antenna of the patch.

Further preferred, the physiological parameter measuring system can comprise at least one electronic conditioning unit, in particular one electronic conditioning unit per antenna patch, connected to an antenna patch and configured to supply a stimulus signal to the antenna patch and to receive a response signal received in turn by the antenna patch from the antenna of the contact lens. For instance the stimulus signal can be a sine wave with a varying frequency, thus like a swept sine signal.

Advantageously, the conditioning unit can furthermore be configured to analyse the response signal, in particular in the frequency domain and of the determining phase properties of the response signal. Thereby the influence of the coupling between the contact lens and the antenna patch can be reduced compared to a measurement based on amplitude measurements. Preferably, one takes advantage of changes in the resonance properties of the antenna's passive sensing means of the contact lens to determine the value of the physiological parameter.

Further preferred, in the physiological parameter measuring system, at least one of the at least one electronic conditioning units is connected, in particular wirelessly connected, to a data monitoring unit. The evolution of the physiological parameter can be easily monitored continuously. Preferably, the data monitoring unit can in turn comprise an interface for transmitting the monitored data of the IOP to another electronic device, e.g. over the internet at a distant location.

Further preferred, the electronic conditioning unit/s is/are attached to a branch or the frame of spectacles and each one of the one or two antenna patches is adhered to one glass, in particular on the side of the glasses towards a patient's eye, of the spectacles respectively. Thereby, a compact measuring system, which is easy to wear by a patient, is provided. The data monitoring unit, with its data storage means, can be transported, for instance in a pocket, so that, in the presence of the data monitoring unit, an easily wearable monitoring system is provided. The electronic conditioning unit can be clipped or adhered to the spectacles of a patient.

Further preferred, the adhesive layer can be configured such that the antenna patch is detachable from the glass of the spectacles, preferably without leaving a residue after removal. Thus a patient can use his own glasses as part of the measuring system and remove the antenna patch, once the monitoring has been accomplished.

Preferably, the physiological parameter measuring system can further comprise a filtering unit configured to reduce spurious artifacts from the response signals based on the detection of a simultaneous predetermined change property of the response signal received from the two contact lenses. Thereby, the spurious values can be automatically removed out of the set of measured pressures to improve the accuracy of the measurement. In that case, it is first checked if the response signal is 'normal' by testing it against criteria. This operation is done on each eye individually. If some abnormal behaviour is observed, then the system is configured to check if the response is similar on both eyes. In that case, it can be eye blinking or an eye rotation, for example, the system decides to erase the result and to repeat the measurement.

According to a further preferred variation, the filtering unit can also be configured to improve the signal-to.-noise ration by reducing the contributions of electromagnetic disturbances which can also be eliminated by doing this dual-channel measurement, because they are present on both when occurring. Thus using a bi-ocular measurement one can take advantage of differential measurements.

Preferably the filtering can be performed using digital signal processing after an analog to digital conversion and can therefore offer a high flexibility concerning data treatment.

Further preferred, the two electronic conditioning units can be arranged in a master and slave configuration, in a system using two electronic conditioning units. Using this configuration, one can balance the weight of or on the branches of the spectacles. For instance, one conditioner can comprise the batteries, power management, and a few first part of electronic components, whereas the other one comprises the remaining electronic components, to achieve this balance.

According to a preferred embodiment, the contact lens can be a vision correcting contact lens or the spectacles can have vision correcting glasses. This further improves the user-friendliness of the inventive physiological parameter measuring system, in particular in combination with the patient's own spectacles.

Advantageously, the antenna and the purely passive sensing means can be arranged on both sides of a flexible carrier substrate. Thus without having to use a larger surface the sensitivity of the system can be improved.

In a variant, the antenna and the purely passive sensing means can comprise spires on the on the one side of a flexible carrier substrate and spires on the other side of a flexible carrier substrate, wherein the spires are connected by a via to form a first and second inductance arranged in series. This arrangement corresponds to a simplified yet functional design.

Preferably, an end region of the spires can be enlarged such as to form a capacitor with a predetermined capacitance to thereby adjust the resonant frequency of the LC circuit.

The invention also relates to a method for measuring a physiological parameter, in particular the intraocular eye pressure, comprising the steps of: a). transmitting a stimulus signal from an antenna patch, in particular adhered to the glasses or spectacles, to a purely passive sensing means of a contact lens, b). receiving a response signal from the purely passive sensing means via the antenna patch, and c). determining the physiological parameter based on a resonant frequency and/or phase shift. Using purely passive sensing means, the radiation energy dissipation present around the physiological parameter measuring system can be kept low to reduce any risks related to their presence in the vicinity of a patient's eye. The antenna patch adhered to the glasses allows an improved coupling between the antennas.

Advantageously, the steps a) - c) can be carried out with two antenna patches and two corresponding contact lenses and, furthermore, comprise a filtering step to filter out spurious artifacts, in particular eye blinking or eye rotations leading to a change of the viewing direction, based on a simultaneous predetermined change property in the response signal received from the two contact lenses. By carrying out a bi-ocular measurement, an automatic filtering can be realized to improve the accuracy of the device.

The invention will be described in more detail in the following based on advantageous embodiments described in conjunction with the following Figures:
- Figure 1: illustrates a physiological parameter measuring system according to a first embodiment of the invention,
- Figure 2a: schematically illustrates the structure of a contact lens used in the first embodiment of the invention,
- Figure 2b: illustrates a practical example of the antenna and sensing means,
- Figure 2c: illustrates a second practical example of the antenna and sensing means,
- Figure 3: illustrates a partial 3-dimensional view of one of the antennas according to a second embodiment,
- Figure 4: illustrates the electrical equivalent circuit,
- Figure 5: illustrates a method according to a third embodiment,
- Figure 6: illustrates a resonance measurement using a practical example according to the invention, and
- Figure 7: illustrates a fourth embodiment according to the invention, wherein the physiological parameter measuring system comprises, in addition, a filtering unit.

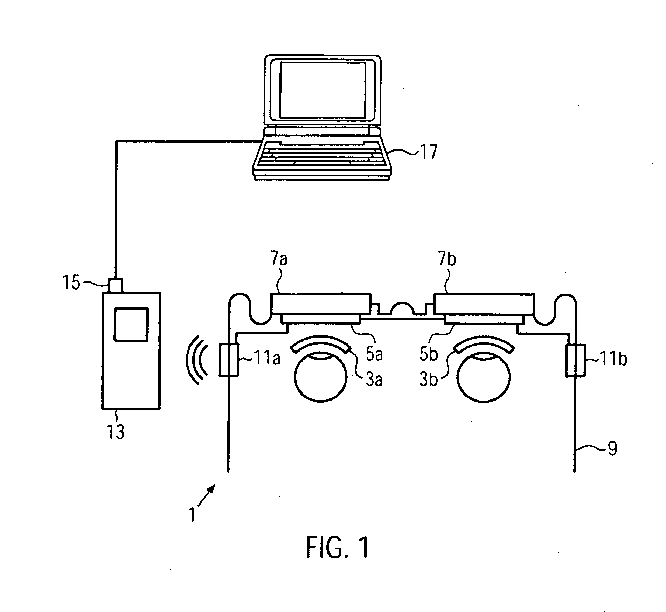

Figure 1 illustrates a physiological parameter measuring system 1 according to a first embodiment of the invention. The physiological parameter measuring system 1 comprises a first and second contact lens 3a, 3b and a first and second antenna patch 5a, 5b adhered to the glasses 7a, 7b of a pair of spectacles 9, on the side of the of the glasses towards a patient's eye. The antenna patches 5a, 5b are connected to a first and second electronic conditioning unit 11 a, 11b, which are connected to each other. Furthermore, one of the electronic conditioning units, in this embodiment 11a, is configured to communicate with a data monitoring unit 13, e.g. using a wireless technology, Wifi, Bluetooth, Zigbee, ISM etc. The data monitoring unit 13 further comprises a data interface 15 via which the monitored data can be transmitted to an external device, for instance a computer 17, e.g. using a wired or wireless communication line.

According to a variant, the system 1 can only comprise one electronic condition unit being connected to both antenna patches 7a, 7b.

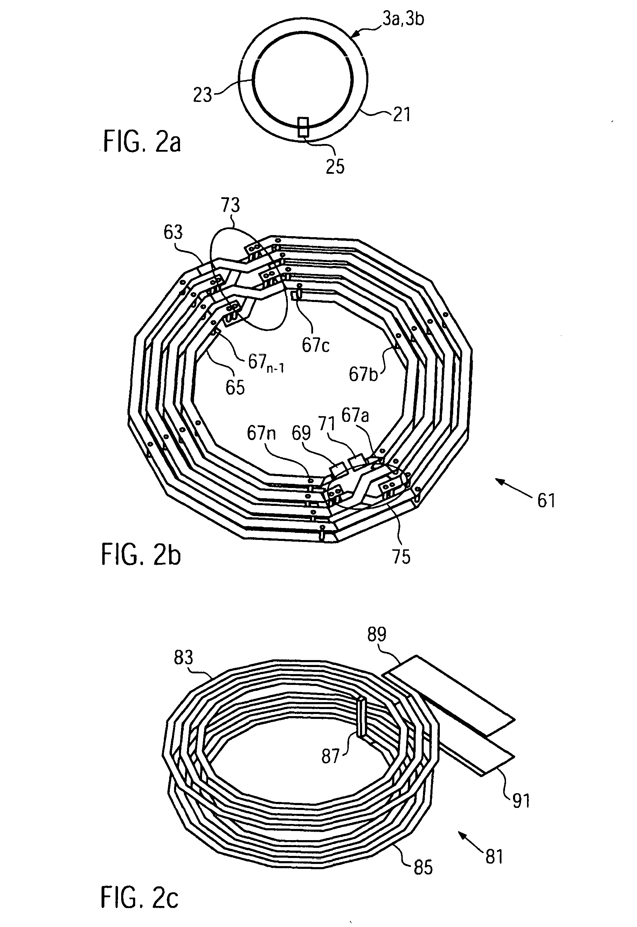

Figure 2a schematically illustrates the structure of each one of contact lenses 3a, 3b. The contact lens comprises a contact lens base 21, comparable to a standard soft contact lens, which can have or not vision correction properties.

In addition, the contact lens 3a, 3b comprises an antenna 23 and a sensing means 25 which, are embedded into the contact lens base 12 to improve the wearing comfort. According to the invention, the sensing means 25 is a fully passive sensing means.

In this embodiment, the purely passive sensing means 25 is a capacitor, e.g. an off the shelf capacitor added to the antenna 23, or an integrated capacitor fabricated at the same time as the antenna loop, or a piezoelectric transducer transforming a mechanical excitation into an electric signal.

Antenna 23 and sensing means 25 effectively build up a passive device corresponding to a LC (inductance + capacitance) circuit. The physiological parameter measurement can then be based on the observation of a frequency drift of the resonance frequency as a function of cornea deformation. The inductance and capacitance are chosen such that a resonance frequency is observed in a frequency range allowed by the national safety regulations, in particular in the so called ISM band 26,957 to 27,283 MHz. To adapt the resonance frequency to such a frequency band, it is possible to adapt the number of spires, e.g. in a rang of about 1 to 5 spires, of the antenna 23 and to adapt the capacitance by using an appropriate additional capacitor in addition to the self capacitance of the antenna loop.

The contact lenses 3a, 3b according to the invention are fabricated in a two step fabrication process. In a practical example according to the invention, first of all, the antenna and sensing means are fabricated. The antenna 23 according to the invention can be formed by a first metal layer, in particular a copper layer, and a second layer on top thereof, in particular out of a biocompatible material like gold, with a spiral form. The first and second layer are provided on one main surface of a carrier substrate, e.g. a flexible material, like for instance polyimide. To improve the sensitivity without having to enlarge the surface covered by the antenna a third layer of copper and a fourth layer of gold can be provided on the opposing other main surface of the carrier substrate, One pair of layers has a thickness in a range of about 20 to 40 µm whereas the flexible carrier substrate typically has a thickness in the range of 20 to 30µm. The antenna 23 is connected to the capacitor (off the shelf or fabricated at the same time than the antenna) or piezoelectric transducer of the sensing means 25 before being mold in a biocompatible material, e.g. a PDMS (polydimethysiloxane) silicon polymer, serving as contact lens. Instead of layer pairs (first and second on the one hand, and third and fourth on the other hand), the invention can also be realized with only one layer, or one layer on each side of the flexible carrier substrate.

Figure 2b illustrates a first practical example 61 of the LC circuit comprising the antenna and the sensing means. In this first practical example spires 63 and 65 are arranged on the front and backside of a flexible carrier substrate, which is not illustrated in this figure. Electric conductive vias, in particular metallic vias, 67a, 67b, ..., 67n-1, 67n connect the spires 63 on the one side and the spires 65 on the other side along their entire length. This arrangement has the advantage that the effective thickness of the spires is essentially doubled and thus the resistance can be reduced and the quality factor of the circuit enhanced. The design is chosen such that the two connecting parts 69 and 71 are side by side on one surface of the flexible carrier substrate to allow the connection to an additional capacitor. The design of figure 2a has two crossing areas 73 and 75 connecting an essentially semi circularly shaped part on an inner area with an essentially semi circularly shaped part in the subsequent round of the spires so that in the end the spires on one side of the substrate never cross more than one spires on the other side of the carrier substrate.

Figure 2c illustrates a second practical example 81. Again spires 83 and 85 are arranged on the front and back side of the flexible carrier substrate, not illustrated. The spires are connected to each other with a via 87 going through the carrier substrate. The via connects the end of the first inductance with the beginning of the second inductance and both are thus forming two inductances connected in series. Both inductances are wound in the same direction, so that the total inductance is enhanced, without needing more space. Figure 2c furthermore illustrates two electrodes 89 and 91 connected to the beginning of the first inductance 83 and the end of the second inductance 85 thereby forming an integrated capacitance to adapt the resonant frequency to the desired frequency range.

Of course, the integrated capacitance could also be provided in the first practical example, just like the design of the second practical example could be connected to an external capacitance.

With the all passive design, it is possible to embed thin components, like already mentioned in the order of 20 to 40µm per layer pair, in the contact lens, the thickness of which can be kept in the order of 100µm which greatly enhances the wearing comfort compared to the prior art device which due to the presence of the active strain gauge, is thicker, typically at least 150µm corresponding to a thickness at which the wearing comfort for the patient reduces rapidly. In addition, the thinner the lens can be made, the more sensitive the IOP measurement. Indeed, a change in pressure can be better indentified for a thin lens than a thick lens which is more rigid.

The use of a purely passive sensing means has the advantage that, compared to active sensing means, the power consumption in the circuit of the contact lens is greatly reduced, so that radiation and energy dissipating phenomena, which may harm the patient's eye, can be kept at a lower level compared to the prior art using active strain gauges.

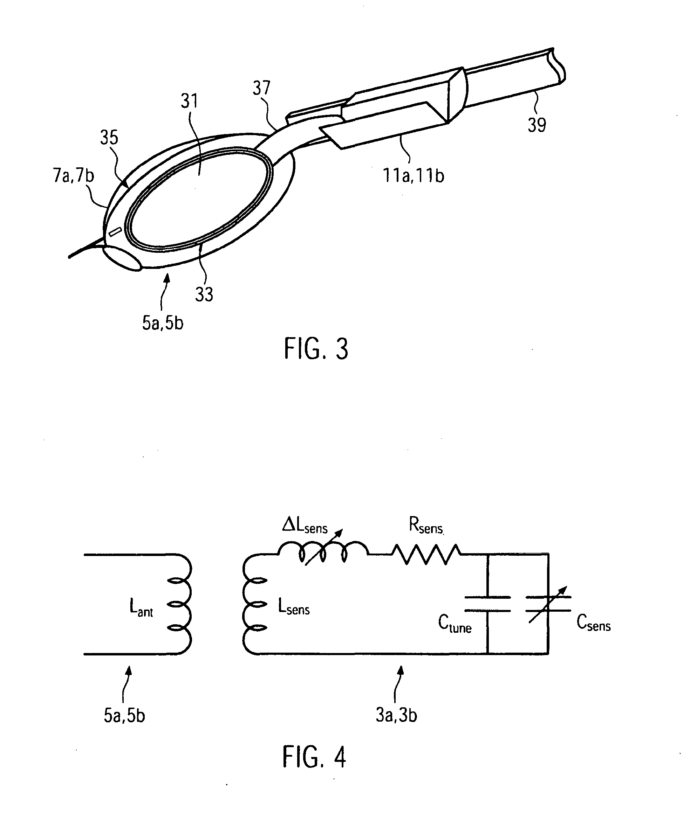

Figure 3 illustrates a partial 3-dimensional view of one of the antenna patches 5a, 5b and the corresponding conditioner 11 a, 11 b.

According to a second embodiment of the invention, the antenna patch 5a, 5b comprises a transparent support layer 31, an antenna loop 33 which, according to a variant, could also be transparent, as well as an adhesive layer 35 used to attach each antenna patch 5a, 5b to one of the glasses 7a, 7b of the spectacles respectively. The adhesive 35 is chosen such that the antenna patch can be removed from the glass 7a, 7b without leaving a trace or without deteriorating the glass 7a, 7b of the spectacles.

This antenna patch 5a, 5b can thus be attached to any glasses with or without vision correction. For instance, the glasses of the spectacles of the patient which he uses in his daily life can be used in the monitoring system 1.

Preferably antenna patches 5a, 5b with various shapes can be provided to be compatible with any form and design of spectacle glasses.

An electric connector 37 links the antenna patch to an electronic conditioning unit 11 a, 11 b being attached or clamped to the branches 39 of the spectacles. In this embodiment, the electrical connection 37 is pinched to the electronic conditioning unit 11 a, 11 b.

Figure 4 illustrates the electrical equivalent circuit of the contact lens 3a, 3b and the antenna patch 5a, 5b. The antenna loop 33 corresponds to the antenna inductance Lₐₙₜ, the antenna 23 to the sensing inductance Lₛₑₙₛ also representing a varying part ΔLₛₑₙₛ, the pressure dependant capacitance changing as a function of contact lens deformation as a consequence of the cornea deformation corresponds to Cₛₑₙₛ, and the eventually present additional capacitance to tune the resonant frequency to the desired value corresponds to Cₜᵤₙₑ. The resistance of the overall circuit design is represented by Rₛₑₙₛ.

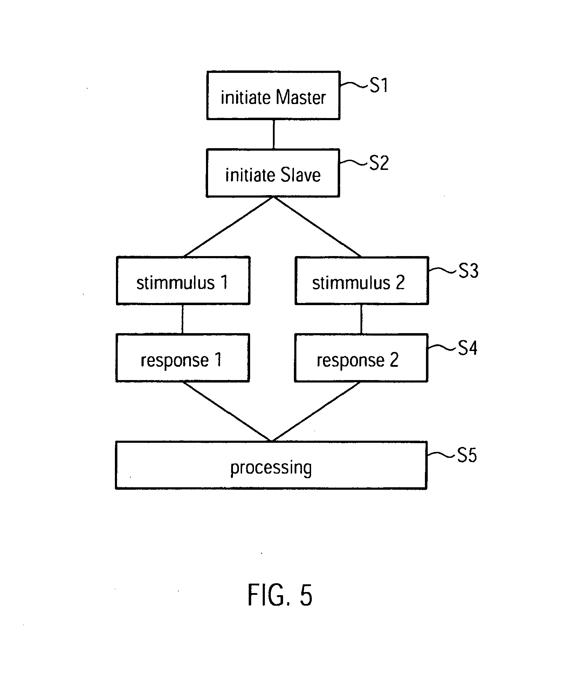

The inventive physiological parameter measuring system as illustrated in Figures 1 - 3 is used according to the following inventive method according to a third embodiment to measure a physiological parameter, in particular the intraocular eye pressure. The method is illustrated in Figure 5.

The electronic conditioning unit 11a, serving as the master electronic conditioning unit, transmits a signal to initiate the slave electronic conditioning unit (Step S2). Subsequently stimulus signals are transmitted via the antennas of the antenna patches 5a, 5b inductively coupled to the antennas 23 of the contact lenses 3a, 3b (Step S3). The contact lenses 3a, 3b then in turn transmit their response signals from the sensing means 25 in each one of the contact lenses 3a, 3b back to the antenna patch (Step S4) so that the response signal can be received by the electronic conditioning unit 11 a, 11 b for further processing (Step S5), either within one or both of the electronic conditioning units 11 a, 11 b or the data monitoring unit 13. The signals in the data monitoring unit 13 are treated in a programmable integrated circuit such as a DSP (Digital Signal Processor), or an FPGA (Field Programmable Gate Array) so that the system 1 is fully programmable with respect to the type of stimulus, the type of pre and post-processing, and thus adaptable to any special demand.

The physiological parameter measurement and here in particular the IOP measurement with the system 1 according to the invention is based on the following method. By combining the antenna patch's excitation voltage with the current going though the antenna loop 33, it is possible to compute the mathematically complex impedance seen from the antenna. A complex impedance Z (with Z = α+jβ) is characterized by a modulus (M) and a phase (Φ). The resonance frequency of the LC circuit of the antenna 23 and the sensing means 25 shifts with IOP variations and the impedance phase (Φ(Z)) goes through a minima at the resonance frequency of the LC circuit on the lens 3a, 3b. Doing a frequency sweep on the antenna patch 5a, 5b thus permits to find out the phase minima and thus the frequency offset due to the IOP variations. The frequency shifts information are transmitted to the data monitoring unit 13 which, after validation of the measurement, is computed into IOP variations by taking into account cornea thickness information, measured on the patient just before this one is equipped with device. The invention thus gives access to the pressure variations which represent an important aspect for glaucoma patients.

The use of the frequency based measurement has the advantage that it is non-sensitive to the amplitude of the signal and consequently also independent of the distance between the glasses of the spectacles and the contact lens 3a. Nevertheless using the transparent antenna patches 5a, 5b, the antenna loop 33 can be placed right in front of the contact lens 3a and 3b. Thus the coupling between the two circuits can be optimized while at the same time wearing comfort can be kept high.

In the embodiment as illustrated in Figures 1 - 3, two contact lenses, two antenna patches and two conditioning units are used, however, without departing from the inventive idea of using only a passive device in the contact lens, the physiological parameter measuring system could be further simplified by only using one contact lens, one antenna patch and one conditioning unit.

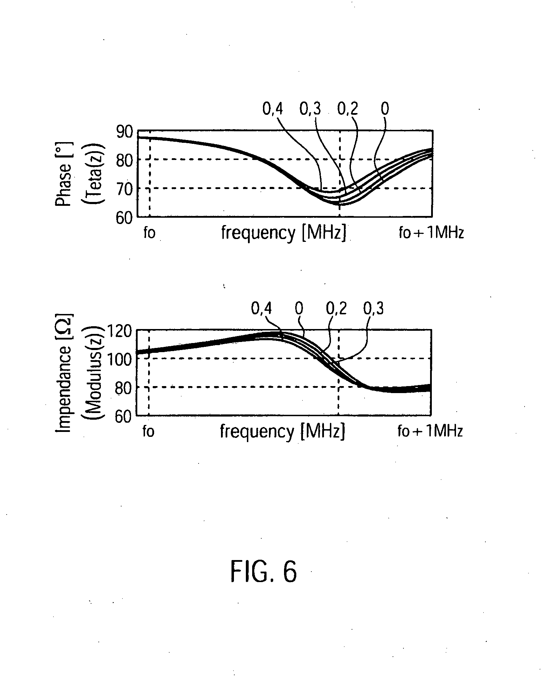

Figure 6 illustrates the results of a frequency and phase measurement using a contact lens with an antenna 23 like illustrated in Figure 2b. To obtain a resonant frequency in the desired range an additional capacitance was added, here an MLCC (Multi-Layer Ceramic Capacitor) type capacitor.

The measurement results illustrated in Figure 6 were obtained by placing the contact lens 3a on an enucleated pork eye with the antenna patch 5a positioned 1,5 cm above the lens. The pressure in the eye was varied by injecting a fluid. In this practical example the four curves correspond to Oml air injected (about 20mmHg IOP), O,2ml air injected (about 70mmHg IOP), 0,3 ml air injected (about 85mmHg IOP) and 0,4 ml air injected (about 90mmHg IOP). Whereas the upper figure illustrates the change in the impedance, the lower figure illustrates the change in phase for IOP pressure changes in the order of 70mmHg a frequency shift of about 77KHz was observed.

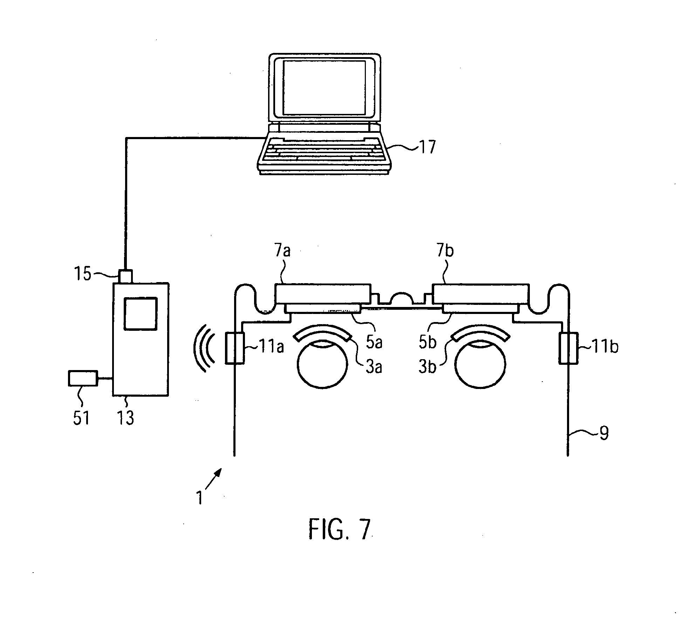

Figure 7 illustrates a fourth embodiment according to the invention, wherein the physiological parameter measuring system 41 comprises, in addition, a filtering unit 43 which is configured to remove spurious artifacts which are observed in the measured signals and which can be due to eye blinking or a change of the viewing direction which lead to predetermined changes in the signals which can be removed by the filtering unit 43 to improve accuracy of the system. As eye blinking and change of viewing direction is typically a synchronized event between the left and the right eye, the observation of the predetermined change in the signal simultaneously in both signal channels is used to trigger a filtering event. To improve the signal-to-noise ration, electromagnetic disturbances of the signals can also be eliminated in the filtering unit based on the dual-channel measurement, as they are present in both response signals when occurring. In this embodiment, the filtering is based on digital signal processing. The filtering unit 43 of this embodiment is embedded in the data monitoring unit 13. As mentioned above, it is easy to detect a simultaneous event on both channels (e.g., same shape in the spectrum).

## Claims

1. Physiological parameter measuring system comprising one or two contact lenses, wherein each contact lens comprises an antenna (23) and a purely passive sensing means (25), wherein
the antenna (23) and the purely passive sensing means (25) comprises spires (63) on the one side of a flexible carrier substrate and spires (65) on the other side of the flexible carrier substrate, **characterized in that** the spires are connected via a plurality of vias (67a - 67n) with each other.

2. Physiological parameter measuring system according to claim 1, wherein the passive sensing means (25) comprises a capacitor or a transducer, in particular a piezoelectric transducer.

3. Physiological parameter measuring system according to claim 1 or 2, wherein the physiological parameter is at least one of intra ocular eye pressure and glucose.

4. Physiological parameter measuring system according to one of claims 1 to 3, wherein the vias (67a -67n) are electric conductive vias for reducing the resistivity.

5. Physiological parameter measuring system according to one of claims 1 to 4, wherein the vias (67a-67n) are arranged over the entire length of the spires.

6. Physiological parameter measuring system according to one of claims 1 to 5, further comprising one or two antenna patches external to the one or two contact lenses.

7. Physiological parameter measuring system according to claim 6, wherein an antenna patch comprises a transparent, in particular flexible, support layer and an adhesive layer.

8. Physiological parameter measuring system according to claim 6 or 7, further comprising at least one electronic conditioning unit, in particular one electronic conditioning unit per antenna patch, connected to an antenna patch and configured to supply a stimulus signal to the antenna patch and to receive a response signal received in turn by the antenna patch from the antenna of the contact lens.

9. Physiological parameter measuring system according to claim 8, wherein the conditioning unit is furthermore configured to analyse the response signal, in particular in the frequency domain and/or by determining phase properties of the response signal.

10. Physiological parameter measuring system according to claim 8 or 9, wherein at least one of the at least one electronic conditioning units is connected, in particular wirelessly connected, to a data monitoring unit.

11. Physiological parameter measuring system according to one of claims 8 to 10, wherein the electronic conditioning unit/s is/are attached to a branch or the frame of spectacles and each one of the one or two antenna patches is adhered to one glass of the spectacles respectively.

12. Physiological parameter measuring system according to claim 11, wherein the adhesive layer is configured such that the antenna patch is detachable from the glass, preferably without leaving a residue after removal.

13. Physiological parameter measuring system according to one of claims 8 to 11, further comprising a filtering unit configured to reduce spurious artefacts from the response signals based on eye blinking or a change of viewing direction, based on the detection of a simultaneous predetermined change property of the response signal received from the two contact lenses.

14. Physiological parameter measuring system according to one of claims 8 to 12, wherein, in the presence of the electronic conditioning units, the two electronic conditioning units are arranged in a master and slave configuration.

15. Physiological parameter measuring system according to one of claims 1 to 14, wherein the contact lens is a vision correcting contact lens or the spectacles have vision correcting glasses.

16. Physiological parameter measuring system according to one of claims 1 to 15, wherein the antenna and the purely passive sensing means are arranged on both sides of a flexible carrier substrate.

17. Method for measuring a physiological parameter, in particular the intra ocular eye pressure, comprising the steps of:
transmitting a stimulus signal from an antenna patch, adhered to the glasses of spectacles to a purely passive sensing means of a contact lens,
receiving a response signal from the purely passive sensing means via the antenna patch, and
determining the physiological parameter based on a resonant frequency and/or phase shift,
wherein the antenna patch and the purely passive sensing means are in accordance with the physiological parameter measuring system according to one of claims 1 to 16.

18. Method according to claim 17, wherein the steps a) to c) are carried out with two antenna patches and two corresponding contact lenses and further more comprising a filtering step to filter out spurious artefacts, in particular eye blinking or eye rotations, based on a simultaneous predetermined change property in the response signal received from the two contact lenses.

## Patentansprüche

1. System zum Messen eines physiologischen Parameters, das eine oder zwei Kontaktlinse/n umfasst, wobei jede Kontaktlinse eine Antenne (23) sowie eine rein passive Erfassungseinrichtung (25) umfasst, und
die Antenne (23) und die rein passive Erfassungseinrichtung (25) Windungen (63) an der einen Seite eines flexiblen Trägersubstrats sowie Windungen (65) an der anderen Seite des flexiblen Trägersubstrats umfassen, **dadurch gekennzeichnet, dass** die Windungen über eine Vielzahl von Kontaktlöchern (67a-67n) miteinander verbunden sind.

2. System zum Messen eines physiologischen Parameters nach Anspruch 1, wobei die passive Erfassungseinrichtung (25) einen Kondensator oder einen Wandler, insbesondere einen piezoelektrischen Wandler, umfasst.

3. System zum Messen eines physiologischen Parameters nach Anspruch 1 oder 2, wobei der physiologische Parameter intraokularer Augendruck oder/und Glukose ist.

4. System zum Messen eines physiologischen Parameters nach einem der Ansprüche 1 bis 3, wobei die Kontaktlöcher (67a-67n) elektrisch leitende Kontaktlöcher zum Verringern des spezifischen Widerstandes sind.

5. System zum Messen eines physiologischen Parameters nach einem der Ansprüche 1 bis 4, wobei die Kontaktlöcher (67a-67n) über die gesamte Länge der Windungen angeordnet sind.

6. System zum Messen eines physiologischen Parameters nach einem der Ansprüche 1 bis 5, das des Weiteren eine oder zwei Antennenfläche/n umfasst, die sich außerhalb der einen oder zwei Kontaktlinse/n befindet/befinden.

7. System zum Messen eines physiologischen Parameters nach Anspruch 6, wobei die Antennenfläche eine transparente, insbesondere flexible, Trageschicht und eine Klebeschicht umfasst.

8. System zum Messen eines physiologischen Parameters nach Anspruch 6 oder 7, das des Weiteren wenigstens eine elektronische Aufbereitungseinheit, insbesondere eine elektronische Aufbereitungseinheit pro Antennenfläche, enthält, die mit einer Antennenfläche verbunden und so eingerichtet ist, dass sie der Antennenfläche ein Anregungssignal zuführt und ein Antwortsignal empfängt, das die Antennenfläche ihrerseits von der Antenne der Kontaktlinse empfängt.

9. System zum Messen eines physiologischen Parameters nach Anspruch 8, wobei die Aufbereitungseinheit des Weiteren so eingerichtet ist, dass sie das Antwortsignal, insbesondere in der Frequenzdomäne, analysiert, und/oder Phaseneigenschaften des Antwortsignals bestimmt.

10. System zum Messen eines physiologischen Parameters nach Anspruch 8 oder 9, wobei wenigstens eine von der wenigstens einen elektronischen Aufbereitungseinheit mit einer Daten-Überwachungseinheit verbunden, insbesondere drahtlos verbunden, ist.

11. System zum Messen eines physiologischen Parameters nach einem der Ansprüche 8 bis 10, wobei die elektronische Aufbereitungseinheit/en an einem Bügel oder dem Rahmen einer Brille angebracht ist/sind und jede von der einen oder den zwei Antennenfläche/n jeweils an einem Glas der Brille angeklebt ist.

12. System zum Messen eines physiologischen Parameters nach Anspruch 11, wobei die Klebeschicht so eingerichtet ist, dass die Antennenfläche, insbesondere ohne Zurückbleiben von Rückständen nach Entfernung von dem Glas gelöst werden kann,.

13. System zum Messen eines physiologischen Parameters nach einem der Ansprüche 8 bis 11, das des Weiteren eine Filtereinheit umfasst, die so eingerichtet ist, dass sie auf Basis der Erfassung einer simultanen vorgegebenen Änderungseigenschaft des von den zwei Kontaktlinsen empfangenen Antwortsignals störende Artefakte in den Antwortsignalen reduziert, die auf Augenzwinkern oder einer Änderung der Blickrichtung beruhen.

14. System zum Messen eines physiologischen Parameters nach einem der Ansprüche 8 bis 12, wobei beim Vorhandensein der elektronischen Aufbereitungseinheiten die zwei elektronischen Aufbereitungseinheiten in einer Master-Slave-Konfiguration angeordnet sind.

15. System zum Messen eines physiologischen Parameters nach einem der Ansprüche 1 bis 14, wobei die Kontaktlinse eine Sehkorrekturlinse ist oder die Brille Sehkorrekturgläser aufweist.

16. System zum Messen eines physiologischen Parameters nach einem der Ansprüche 1 bis 15, wobei die Antenne und die rein passive Erfassungseinrichtung an beiden Seiten eines flexiblen Trägersubstrats angeordnet sind.

17. Verfahren zum Messen eines physiologischen Parameters, insbesondere des intraokularen Augendrucks, das die folgenden Schritte umfasst:
Senden eines Anregungssignals von einer Antennenfläche, die an den Gläsern einer Brille angeklebt ist, zu einer rein passiven Erfassungseinrichtung einer Kontaktlinse,
Empfangen eines Antwortsignals von der rein passiven Erfassungseinrichtung über die Antennenfläche, und
Bestimmen des physiologischen Parameters auf Basis einer Resonanzfrequenz und/oder Phasenverschiebung,
wobei die Antennenfläche und die rein passive Erfassungseinrichtung dem System zum Messen eines physiologischen Parameters nach einem der Ansprüche 1 bis 16 entsprechen.

18. Verfahren nach Anspruch 17, wobei die Schritte a) bis c) mit zwei Antennenflächen und zwei entsprechenden Kontaktlinsen ausgeführt werden, und das Verfahren des Weiteren einen Filter-Schritt umfasst, in dem auf Basis einer simultanen vorgegebenen Änderungseigenschaft des von den zwei Kontaktlinsen empfangenen Antwortsignals störende Artefakte, insbesondere Augenzwinkern oder Augendrehungen, herausgefiltert werden.

## Revendications

1. Système de mesure d'un paramètre physiologique comprenant une ou deux lentilles de contact, dans lequel chaque lentille de contact comprend une antenne (23) et un moyen de détection purement passif (25), dans lequel l'antenne (23) et le moyen de détection purement passif (25) comprennent des spires (63) sur un côté d'un substrat porteur flexible et des spires (65) sur l'autre côté du substrat porteur flexible,
**caractérisé en ce que**
les spires sont connectées entre elles par une pluralité de vias (67a-67n).

2. Système de mesure d'un paramètre physiologique selon la revendication 1, dans lequel le moyen de détection passif (25) comprend un condensateur ou un transducteur, en particulier un transducteur piézoélectrique.

3. Système de mesure d'un paramètre physiologique selon la revendication 1 ou 2, dans lequel le paramètre physiologique est au moins un paramètre parmi la pression intra-oculaire et le taux de glucose.

4. Système de mesure d'un paramètre physiologique selon l'une des revendications 1 à 3, dans lequel les vias (67a -67n) sont des vias conducteurs d'électricité pour réduire la résistivité.

5. Système de mesure d'un paramètre physiologique selon l'une des revendications 1 à 4, dans lequel les vias (67a-67n) sont agencés sur toute la longueur des spires.

6. Système de mesure d'un paramètre physiologique selon l'une des revendications 1 à 5, comprenant en outre une ou deux antennes planaires externes auxdites une ou deux lentilles de contact.

7. Système de mesure d'un paramètre physiologique selon la revendication 6, dans lequel une antenne planaire comprend une couche de support transparente, en particulier flexible, et une couche adhésive.

8. Système de mesure d'un paramètre physiologique selon la revendication 6 ou 7, comprenant en outre au moins une unité de conditionnement électronique, en particulier une unité de conditionnement électronique par antenne planaire, connectée à une antenne planaire et configurée pour fournir un signal d'excitation à l'antenne planaire et pour recevoir un signal de réponse reçu à son tour par l'antenne planaire en provenance de l'antenne de la lentille de contact.

9. Système de mesure d'un paramètre physiologique selon la revendication 8, dans lequel l'unité de conditionnement est en outre configurée pour analyser le signal de réponse, en particulier dans le domaine de fréquence, et/ou en déterminant des propriétés de phase du signal de réponse.

10. Système de mesure d'un paramètre physiologique selon la revendication 8 ou 9, dans lequel au moins une desdites au moins une unités de conditionnement électronique est connectée, en particulier connectée sans fil, à une unité de surveillance de données.

11. Système de mesure d'un paramètre physiologique selon l'une des revendications 8 à 10, dans lequel la ou les unités de conditionnement électronique sont attachées à une branche ou à la monture de lunettes, et chacune desdites une ou deux antennes planaires est respectivement adhérée à un verre des lunettes.

12. Système de mesure d'un paramètre physiologique selon la revendication 11, dans lequel la couche adhésive est configurée de telle sorte que l'antenne planaire est détachable du verre, de préférence sans laisser de résidu après son détachement.

13. Système de mesure d'un paramètre physiologique selon l'une des revendications 8 à 11, comprenant en outre une unité de filtrage configurée pour réduire des artéfacts aberrants dans les signaux de réponse sur base d'un clignement de l'oeil ou d'un changement de direction du regard, sur base de la détection d'une propriété de changement prédéterminé simultané du signal de réponse reçu des deux lentilles de contact.

14. Système de mesure d'un paramètre physiologique selon l'une des revendications 8 à 12, dans lequel, en présence des unités de conditionnement électronique, les deux unités de conditionnement électronique sont agencées dans une configuration maître-esclave.

15. Système de mesure d'un paramètre physiologique selon l'une des revendications 1 à 14, dans lequel la lentille de contact est une lentille de contact de correction visuelle, ou dans lequel les lunettes portent des verres correcteurs de vision.

16. Système de mesure d'un paramètre physiologique selon l'une des revendications 1 à 15, dans lequel l'antenne et le moyen de détection purement passif sont agencés des deux côtés d'un substrat porteur flexible.

17. Procédé pour mesurer un paramètre physiologique, en particulier la pression intra-oculaire, comprenant les étapes suivantes :
transmission d'un signal d'excitation provenant d'une antenne planaire, adhérée aux verres des lunettes, à un moyen de détection purement passif d'une lentille de contact,
réception d'un signal de réponse provenant du moyen de détection purement passif via l'antenne planaire, et
détermination du paramètre physiologique sur base d'une fréquence de résonance et/ou d'un décalage de phase,
dans lequel l'antenne planaire et le moyen de détection purement passif sont conformes au système de mesure d'un paramètre physiologique selon l'une des revendications 1 à 16.

18. Procédé selon la revendication 17, dans lequel les étapes a) à c) sont mises en oeuvre avec deux antennes planaires et deux lentilles de contact correspondantes, et comprenant en outre une étape de filtrage pour filtrer des artéfacts aberrants, en particulier un clignement ou une rotation de l'oeil, sur base d'une propriété de changement prédéterminé simultané du signal de réponse reçu des deux lentilles de contact.
